# EUROPEAN PATENT APPLICATION

(11) **EP 4 102 511 A1**
(43) Date of publication of application: **14.12.2022**
(21) Application number: 21305807.6
(22) Date of filing: 11.06.2021
(51) Int. Cl.: G16H 20/10, A61M 5/142

(54) **METHOD FOR DETERMINING AN EFFECT OF A DRUG ON A PATIENT WITH A SPECIFIC DOSE REGIMEN**

(71) Applicant: Lixoft, 92160 Antony (FR)
(72) Inventor: CHAUVIN, Jonathan, 75017 Paris (FR); AYRAL, Géraldine, 92160 Antony (FR); KALIFA, Jérôme, 92160 Antony (FR)
(74) Representative: Plasseraud IP

(57) **Abstract**

The disclosure relates to a method for determining an effect of a drug on a patient, with at least one specific dose regimen.

A population pharmacological model describing a variability in drug exposure within a patient population receiving known doses of said drug is obtained (S1).

For at least one parameter relating to a source of said variability within the patient population, a plurality of values of said parameter is sampled from a distribution conditional on individual patient characteristics and/or at least one measurement related to a drug exposure of the at least one patient. By using the population pharmacological model, a corresponding drug exposure with given dose regimens is calculated (S4), for sets of the sampled simulated values.

The effect of the drug on a given patient, with at least one specific dose regimen, is determined (S10) based at least on the calculated drug exposures.

The disclosure further relates to a corresponding computer program and processing circuit.

## Description

### DOMAIN

The disclosure belongs to the field of bioinformatics. It generally relates to pharmacokinetics or pharmacodynamics.

In particular, there are disclosed a method for determining an information related to a drug on at least one patient, with at least one specific dose regimen, a corresponding computer program and a corresponding processing circuit.

### BACKGROUND

Therapeutic drug monitoring (TDM) is a branch of clinical chemistry and clinical pharmacology that specializes in the measurement of medication levels, for example in blood, tissues, or organs. Its main focus is on drugs with a narrow therapeutic range, i.e. drugs that can easily be under- or overdosed. TDM aimed at improving patient care by individually adjusting the dose of drugs for which clinical experience or clinical trials have shown it improved outcome in the general or special populations. It can be based on a a priori pharmacogenetic, demographic and clinical information, and/or on the a posteriori measurement of concentrations of drugs (pharmacokinetic monitoring) or biological surrogate or end-point markers of effect (pharmacodynamic monitoring).

There are numerous variables that influence the interpretation of drug concentration data: time, route and dose of drug given, time of sampling, handling and storage conditions, precision and accuracy of the analytical method, validity of pharmacokinetic models and assumptions, co-medications and, last but not least, clinical status of the patient (i.e. disease, renal/hepatic status, biologic tolerance to drug therapy, etc.).

Many different professionals (physicians, clinical pharmacists, nurses, medical laboratory scientists, etc.) are involved with the various elements of drug concentration monitoring, which is a truly multidisciplinary process. Because failure to properly carry out any one of the components can severely affect the usefulness of using drug concentrations to optimize therapy, an organized approach to the overall process is critical.

To perform dose individualization, there is a need for determining or predicting, rationally and accurately, an effect of a drug on a specific patient, even though multiple patients receiving the same doses of a drug may exhibit variability in drug exposure.

### SUMMARY

The invention is defined by the appended independent claims. Additional features and advantages of the concepts herein disclosed are set forth in the description which follows.

The present disclosure aims at improving the situation.

To this end, the present disclosure describes a method for determining an effect of a drug on at least one patient, with at least one specific dose regimen, the method comprising:
- obtaining a population pharmacological model, said model describing a variability in drug exposure within a patient population receiving known doses of said drug,
- for at least one parameter relating to a source of said variability within the patient population, sampling a plurality of values of said parameter from a distribution conditional on individual patient characteristics and/or at least one measurement related to a drug exposure of the at least one patient,
- forming sets of values, one set of values comprising one sampled value of each of said parameter,
- by using the population pharmacological model, calculating, for each set of values, a corresponding drug exposure with given dose regimens, and
- determining the effect of the drug on at least one patient, with at least one specific dose regimen, based at least on the calculated drug exposures.

In the context of the invention:
- the "at least one patient" designates a subset of the whole patient population covered by the population pharmacological model, this subset being also referred to, hereafter, as forming a population of patients under consideration,
- the "parameters" are pharmacokinetic or pharmacodynamic data, such as apparent volume of distribution or elimination rate constant, which collectively relate to the variability in drug exposure within the patient population,
- the "individual patient characteristics" are descriptive input data, such as weight or age of individuals, which values allow determining corresponding values of the above parameters through correlations or computations.

Each set of values represents a plausible representation of a corresponding patient, or individual. For each given individual, a corresponding plurality of sets of values are formed Those sets of values are statistically representative of the uncertainty of the individual's parameters. Due to how the sets of values are formed, the sets of values of all individuals are, collectively, statistically representative of a patient population defined by the population pharmacological model in terms of variability in drug exposure when receiving known doses of the drug. As a matter of fact, the sets of values of all individuals are also, collectively, statistically representative of the patient population in terms of variability in one or more effects of the drug when receiving known doses of the drug.

Therefore, by calculating for each set of values the corresponding drug exposure with a series of tested given dose regimens, the result of the calculations is a set of indications of the individual drug exposures that would be caused if each of these tested given dose regimens, respectively, was actually applied to each patient. Collectively, the individual drug exposures are indications of drug exposures that would be caused if each of these tested given dose regimens, respectively, was actually applied to the population of patients under consideration.

Such set of indications is a key input for determining the effect of the drug on a patient with a specific dose regimen.

For example, the individual drug exposures may be translated into an occurrence, or a level of magnitude, of a therapeutic effect or of an undesirable effect. Considering a given dose regimen, it is then possible to determine, for example:
- a frequency of occurrence of an undesirable effect within the patient population, or
- a mean level of magnitude of a therapeutic effect within a subgroup of the patient population having similar patient profiles, or
- an expected level of magnitude, possibly associated to an uncertainty window, of a therapeutic effect for a specific patient, or
- a projected level of risk of an undesirable effect for a specific patient.

As a result, the calculated drug exposures allow for a comparison of the tested given dose regimens, in order to select, among the given dose regimens, a specific dose regimen that provides the best risk-benefit ratio for a patient.

Alternately, it is also possible to interpolate the calculated drug exposures for different tested given dose regimens, and as a result determine an interpolated specific dose regimen that provides an even better risk-benefit ratio for a patient than any of the tested given dose regimens.

The above method may find various applications, for example the determined effect of the drug on a patient with a specific dose regimen is valuable information for a physician seeking to determine an initial dose regimen for a patient or seeking to modify a dose regimen that a patient is currently submitted to.

Optionally, the patient being submitted to a former dose regimen, the method further comprises obtaining at least one measurement related to a drug exposure of the patient with the former dose regimen, and determining the effect of the drug on the patient is further based on said measurement.

In general, when a patient already receives known doses of the drug, there are various ways to determine how well the patient tolerates the drug at this dose regimen. Such determination may be based, for example, on various measurements such as blood examination or the like, on clinical or physiological observations performed by a physician, on feedback from the patient, or on a combination of such possible inputs. Then in combination with the calculated drug exposures, it is possible to adapt the former dose regimen and perform dose individualization. For example, the former dose regimen may be associated, according to the calculated drug exposures, to a low risk of an undesirable effect with a moderate to high intensity. If, for instance, said undesirable effect is indeed present for the patient at a high intensity, then the calculated drug exposures may be used to determine a safer dose regimen.

The same principle applies for determining a more beneficial dose regimen for the patient in case a desired, therapeutic, effect is not obtained, or insufficiently obtained, in the patient with the former dose regimen.

Optionally, determining the effect of the drug comprises comparing the calculated drug exposures to at least one exposure threshold.

For example, the calculated drug exposures may each be compared to a lower boundary and to a higher boundary forming a therapeutic window.

Such comparison allows determining, for each tested given dose regimen, a corresponding proportion of the simulated individual drug exposures for which the resulting drug exposure falls within the therapeutic window. Such comparison may further be used to determine, for each tested given dose regimen, a corresponding indication of a probability that the resulting drug exposure for the patient would match the therapeutic window.

Optionally, the method further comprises linking each calculated drug exposure to a corresponding level of magnitude of said effect of the drug, and determining the effect of the drug, with given dose regimens, on said patient, is based on the linked levels of magnitude.

For example, considering a tested given dose regimen, the levels of magnitude linked to the calculated drug exposures may be used to predict, for a patient, an expected level of magnitude of said effect of the drug with said tested given dose regimen. This expected level of magnitude may further be associated to an uncertainty window.

Optionally, determining the effect of the drug comprises determining a score representative of said effect based on the calculated drug exposures.

Partial scores may indicate, for example, an average level of magnitude, or a frequency of occurrence, of a given positive or negative effect of the drug among the patient population with a given dose regimen. A global score, for that given dose regimen, may be an aggregate of such partial scores.

Optionally, the patient population is a selected portion of a general population.

This corresponds to filtering a generic patient population model in order to focus, specifically, on the variability in drug exposure within a selected portion of the generic patient population receiving known doses of said drug.

The resulting sets of sampled values represent, specifically, hypothetical patients within the selected portion of the general population.

Possible selection criteria may for example comprise gender, age, weight, blood type, known health problems, medical conditions, allergies, ongoing treatments, pharmacogenomics, and the like.

Therefore, it is possible to first specify a portion of the general patient population which an actual patient belongs to, then form sets of values representing hypothetical patients, all belonging to the specified portion of the general population, then calculate the drug exposures of these hypothetical patients for different dose regimens of a drug, and finally determine the effect of the drug of the actual patient based on the calculated drug exposures.

To obtain the same end result in this matter, focusing on a selected portion of a general patient population requires less computational resources than taking into account the whole general patient population.

Optionally, the drug exposure relates to an instantaneous exposure value.

For example, after receiving a known dose of a drug, the patient may metabolize the drug, resulting in an increasing concentration of the metabolite, up to a maximum instantaneous exposure value after a certain period of time. Then, the metabolite is progressively eliminated by the body. As long as the patient does not receive a further dose of the drug, this elimination results in a decreasing concentration of the metabolite, down to a minimum instantaneous exposure value. The amount of drug received at a time by the patient, and the time interval between two consecutive administrations of the drug to the patient are two important parameters of a dose regimen, that allow controlling the minimum and maximum instantaneous exposure values.

Optionally, the drug exposure relates to an average or an aggregate exposure across a predetermined time interval.

For example, occurrence of some undesirable effects may be associated to an average exposure, or an aggregate, or total, exposure, over a specific time interval exceeding a threshold value.

Optionally, the method further comprises, for at least one parameter relating to a source of said variability within the patient population, using a Markov Chain Monte Carlo approach (one example is a Metropolis Hastings algorithm) applied to the pharmacological model, and sampling the plurality of values of said parameter is based on Markov chains constructed using said Markov Chain Monte Carlo approach.

MCMC methods are a class of algorithms for sampling from probability distributions for which direct sampling is difficult. The output of calculations based on MCMC methods, with moderate computational resources, allow generating hundreds of values representing uncertainty of the parameters relating to sources of the variability in drug exposure for a given individual receiving known doses of a drug, with at least one measurement related to a drug exposure. This sampling approach is equivalent to performing an individual Bayesian estimation taking the population distribution p(ψ) as prior, along with the individual data, and obtaining a conditional distribution p of values of said parameter within the patient population, such conditional distribution being noted (ψ|y), as posterior.

As such, optionally, the method comprises, for at least one parameter relating to a source of said variability within the patient population, using direct sampling from an explicit conditional distribution of values of said parameter within the patient population.

Optionally, the method further comprises, for at least one additional dose regimen:
- by using the population pharmacological model, calculating, for each set of values, a corresponding additional drug exposure with said additional dose regimen, and,
- determining the corresponding effect of the drug, with said additional dose regimen, on the patient based on the calculated additional drug exposures.

This allows comparing the expected effects of the drug on the patient with different possible dose regimens. The result of the comparison may be used as assistance information for determining a suitable dose regimen for the patient.

Optionally, the method further comprises, at least for the given dose regimen, providing the corresponding determined effect in view of determining a personalized dose regimen of the drug for the patient.

For instance, the determined effect may be for example associated to the given dose regimen and stored in a database. The determined effect may also be displayed, possibly along with other determined effects for other tested given dose regimens.

Another aspect of the disclosure is a computer program comprising one or more stored sequences of instructions that is accessible to a processing unit and which, when executed by the processing unit, causes the processing unit to carry out the above method.

Another aspect of the disclosure is a processing circuit equipped with a processing unit operably connected to a memory, the processing circuit being configured to carry out the above method.

### BRIEF DESCRIPTION OF THE DRAWINGS

Figure 1 illustrates a flowchart of a general algorithm of a computer program according to an exemplary embodiment of the invention.
Figure 2 illustrates a processing circuit according to an exemplary embodiment of the invention.

### DETAILED DESCRIPTION

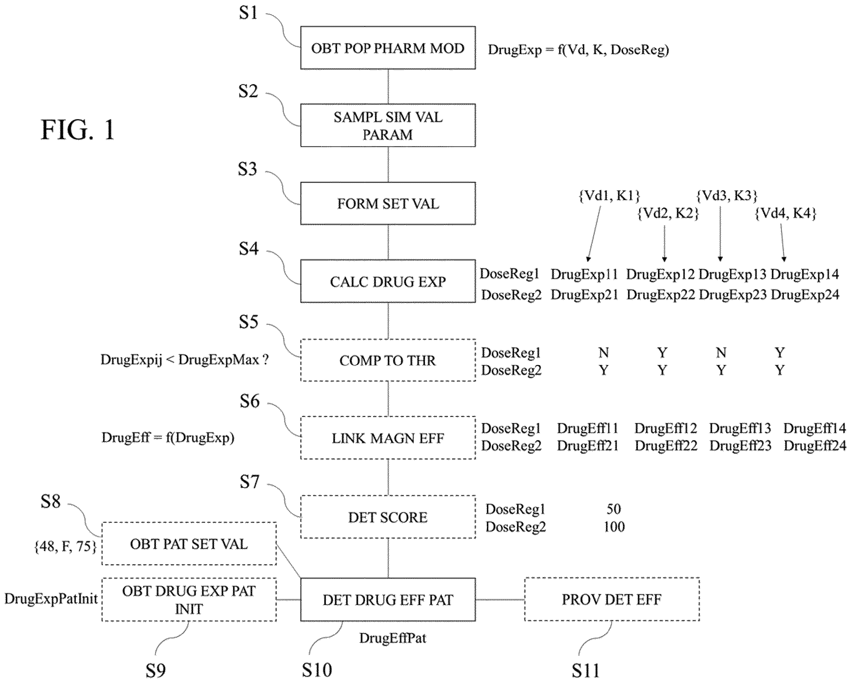

It is referred throughout this section to Figure 1, which depicts a flowchart of a general algorithm of a computer program. When executed by a processor, such computer program causes the processor to carry out a method according to an exemplary embodiment of the invention.

Different modules are described hereinafter, and are to be considered as software and/or hardware functional modules configured so that the processor carries out an instruction of the program, or a set of instructions of the program, specifically relating to the function of the module.

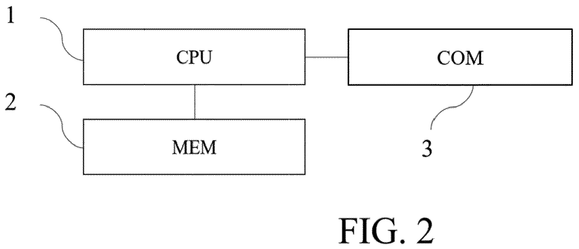

Figure 2 depicts an exemplary processing circuit, comprising a processor (1) operably connected to a memory (2), the processing circuit being configured to carry out the above method. The processing circuit may further comprise a communication interface (3) configured to allow receiving inputs and/or transmitting outputs, whenever applicable. It is for example referred in this section to a human-machine interface, such as a keyboard or a touch screen, which is an example of a communication interface allowing receiving input data. It is also referred to a visual display which is an example of a communication interface allowing transmitting output data, for example in the form of an image or of text. An external drive or a cloud is an example of a input/output communication interface, which may:
- store input data such as a population pharmacological model or values of parameters to be input in the equations of such model, or
- store output data such as a determined drug effect with a dose regimen on a patient.

A population pharmacological model is obtained (S1) by a model retrieving module.

A pharmacological model is associated to a drug and describes the variation with respect to time of a drug concentration, and/or of a drug metabolite concentration, and/or of any effect directly or indirectly induced, following an absorption of a known quantity of the drug. Such an absorption may for example be orally or by injection. Such a model can be described by analytical equations or ordinary differential equations. The use of such parametric models allows describing the full pharmacokinetics and/or pharmacodynamics of the drug.

Population pharmacological models are a variation of generic pharmacological models, for the purpose of taking the variability between individuals into account. The parameters describing a population pharmacological model are not fixed, but rather follow a statistical law to describe a patient population. This allows determining the typical population response to receiving known doses of a drug and quantifying variability in pharmacokinetics and pharmacodynamics of the drug among the population.

Typically, the obtained population pharmacological model is a representation of literature data, which may include, but may not necessarily be limited to, data from the submission report of the considered molecule.

Based on the statistical laws embedded in the population pharmacological model, it is possible to isolate a relevant set of factors, as parameters relating to main sources of variability in drug exposure within the patient population when receiving known doses of the drug.

In the framework of the present document, such a relevant set of parameters is assumed to comprise at least one parameter, possibly two, three or more, to be sampled.

The nature of such parameters depends on the specific use case at hand.

The use case at hand is defined at least based on the obtained population pharmacological model.

For example, considering a first specific drug, age may be identified as a decisive factor on drug exposure of patients submitted to a given dose regimen. On the contrary, considering a second specific drug, it may be observed, or computed, that the drug exposure of patients submitted to a given dose regimen may undergo only normal statistical variations across a large age range. As a result, for the second specific drug, age may be identified as playing little to no influence on drug exposure of patients submitted to said given dose regimen.

The use case at hand may optionally be further defined based on specific information relating to an actual patient. For example, an actual patient may already be receiving the drug, according to a dose regimen (hereafter, the "former dose regimen") and an observation, or a measurement, related to the resulting drug exposure of the actual patient, and/or to one or more resulting effects on the patient, may have been obtained. Such observation or measurement may be used as additional input to target a relevant set of parameters to be sampled.

In a hands-free approach, the nature and number of such parameters may be automatically selected depending on the specific use case at hand, for example based on a principal component analysis.

Alternately, the nature and number of such parameters may be preselected while allowing a user to perform a different selection of parameters, or to expand or reduce the number of selected parameters at will, through use of a human-machine interface. Examples include in particular a keyboard, a mouse, a touch screen, buttons, sliders, use of a speech recognition software, and the like. The nature and number of such parameters may also be fully left to selection by a user, without any preselection.

In Figure 1, it is assumed that, in an exemplary use case, the apparent volume of distribution and the elimination rate constant, which are both pharmacokinetic parameters, may form such a relevant set of parameters. In general, the relevant set of parameters may comprise any type of pharmacological parameters, including pharmacokinetic and/or pharmacodynamic parameters. As a result, in such a use case, the obtained population pharmacological model comprises an equation, or a set of equations, allowing expressing a drug exposure of a patient as a function of indications of apparent volume of distribution, elimination rate constant and dose regimen.

For example:
- apparent volume of distribution and elimination rate constant may each be indicated, for instance, by a specific number or by a range,
- dose regimen may be indicated, for instance, by a frequency of dose administration combined with an amount of drug per administrated dose.

For at least one parameter relating to a source of variability in drug exposure within the patient population when receiving known doses of the drug, a plurality of simulated values of said parameter are sampled (S2) by a sampling module.

Figure 1 shows, in an exemplary use case, a sampling of two parameters:
- numerical values of a first parameter related to an apparent volume of distribution, and
- numerical values of a second parameter related to an elimination rate constant.

As already mentioned, the population model provides the statistical law of each parameter, noted p(ψ), where ψ is the vector of parameters. The conditional distribution corresponds to the statistical law for each individual assuming input values of the patient characteristics and/or at least one measurement related to a drug exposure are known and is noted p(ψ|y), where y relate to the measured patient characteristics and/or measurement related to a drug exposure.

The conditional distribution represents the uncertainty of the individual parameter values for a given individual. It is generally not possible to directly calculate the probability distribution, but it is possible to obtain samples from the distribution using, for example, a Markov-Chain Monte-Carlo procedure (MCMC).

MCMC methods are a class of algorithms for sampling from probability distributions for which direct sampling is difficult. They consist of constructing a stochastic procedure which, in its stationary state, yields draws from the probability distribution of interest.

Among the MCMC class, the Metropolis-Hastings (MH) algorithm has the property of being able to sample probability distributions which can be computed up to a constant. This is generally the case for such conditional distributions, which can then be rewritten as: p(ψ|y)=(p(p|ψ)p(ψ))/(p(y)).

p(y|ψ) is the conditional density function of the data when knowing the individual parameter values. p(y|ψ) can be computed using the model simulation with the individual parameter values and a probability density closed form expression.

p(ψ) is the density function for the individual parameters within the population and can also be computed.

The likelihood p(y) has, generally, no closed form solution but is constant.

In brief, the MH algorithm undergoes numerous iterations. At each iteration, a new individual parameter value is drawn from a proposal distribution for each individual. Then, such new value is accepted with a probability that depends on p(ψ) and p(y|ψ). Finally, after a transition period, the algorithm reaches a stationary state where the accepted values follow the conditional distribution probability p(ψ|y). For the proposal distribution, various different distributions are possible, among which the population distribution, a unidimensional Gaussian random walk, or a multidimensional Gaussian random walk. For the random walks, the variance of the Gaussian may be automatically adapted to reach an optimal acceptance ratio.

As a result, for at least one parameter relating to a source of variability in drug exposure within the patient population when receiving known doses of the drug, a set of n parameter values is obtained by sampling, n being an integer smaller than, or equal to, the total number of iterations of the MH algorithm.

For any specific parameter of any specific individual, these sampled parameter values are noted ψi, with i being a positive integer from 1 to n. The empirical distribution of ψi is representative of the uncertainty of ψ for a given individual. In other words, the sampled parameter values ψi are indicative of an estimation of a conditional distribution of values of said parameter for the individual. The value of n is to be chosen as a compromise between allowing ψi being as representative as possible of the uncertainty of ψ and minimizing the computational requirements. The inventors obtained satisfactory results by setting a predetermined value of n in the range of hundreds to thousands, for example n may be equal to 500.

Sets of the sampled parameter values are formed (S3) by a regrouping module.

The sets of the sampled parameter values comprise one parameter value for each parameter for which a plurality of simulated values have been sampled (S2) by the sampling module.

In the particular case where a plurality of simulated values of a single parameter have been sampled (S2) by the sampling module, then the sets of the sampled parameter values, formed (S3) by the regrouping module, are singletons.

Figure 1 shows, in an exemplary use case, a plurality of sets of values, each set being formed of:
- one numerical value of a first parameter, noted Vd, related to an apparent volume of distribution, and
- one numerical value of a second parameter, noted K, related to an elimination rate constant.

The pharmacokinetic and/or pharmacodynamic individual parameters forming the sets of values may be generated, sampled or computed based on input values of individual patient characteristics.

In an example, individual patient characteristics may comprise:
- one numerical value (in years) of an individual parameter relating to age, as well as
- one binary value (shown as M/F) of an individual parameter relating to gender, and
- one numerical value (in kg) of an individual parameter relating to weight.

Each set of values is representative of a 'simulated patient'. In the example of Figure 1, a 'simulated patient' is a hypothetical patient characterized by their apparent volume of distribution and elimination rate constant.

In an exemplary implementation, sampling (S2) the values of a plurality of parameters may first be performed as a whole, and the sets of the sampled parameter values may be formed (S3), as a whole, only afterwards.

In another exemplary implementation, sampling (S2) the values of a plurality of parameters and forming (S3) the sets of the sampled parameter values may be performed in an integrated manner, by a module having both the functions of the sampling module and of the regrouping module.

Specifically, an intermediate iteration of an algorithm for sampling the values of two parameters and forming the sets of the sampled parameter values may consist in:
- obtaining a plurality of sets of parameter values obtained through previous iterations of the algorithm,
- sampling a new set of parameter values, as a vector of parameters, regrouping a sampled given value per each parameter,
- checking, based on the sampled given values of both parameters and on a known conditional distribution of values of one of these parameters knowing the value of the other parameter, whether the new set would meet a statistical hypothesis with respect to the obtained plurality of sets of parameter values obtained through previous iterations, and
- based on the result of the check, updating the plurality of sets of parameter values with the new set or rejecting the new set.

For example, the abovementioned check may be a comparison with regard to a threshold value, such as a chi-squared test, a t-test or any other type of test known by the skilled person.

Each formed set of parameter values may be stored and used as basis to calculate drug exposures according to different dose regimens based on the population pharmacological model.

Specifically, at least one dose regimen, characterized by an amount of the drug to be administered to a patient as a function of time, may be tested. The amount of the drug may be expressed, for example, as a weight of the drug, as a number of units such as pills containing a known quantity of the drug, as a volume of a solution having a known concentration of the drug, etc. The dose regimen may be expressed, for example, as "3 pills of 400 mg of drug each per day", or as "5 mL injections, at a 50 mg/mL concentration, spaced by a week".

Alternately, a plurality of dose regimens may be tested. These dose regimens may for example be automatically retrieved from a database, or may be manually entered or selected using a human-machine interface.

The tested dose regimens may be predefined taking operational constraints in terms of amount of drug per dose and/or in terms of frequency of administration into account. The possible amount may be related to available forms of the drug, and may correspond to a number of pills if the drug is only available in an oral form. The possible frequency may be based on literature data or be fixed.

A drug exposure calculator module may be configured to calculate (S4), using the population pharmacological model, the drug exposures of each 'simulated patient' with each dose regimen to be tested.

To calculate the drug exposure of a single 'simulated patient' with a single dose regimen, the drug exposure calculator module inputs the specific parameter values from the formed set of sampled parameter values representing said 'simulated patient' as the parameters of the parametric model forming the population pharmacological model.

Then, the drug exposure calculator module may calculate any aspect of the drug exposure of the 'simulated patient', based on the equations of the parametric model with said inputted values, following the administration of each dose of the drug as defined by the dose regimen to be tested.

The drug exposure calculator may calculate, for example, a variation with respect to time of the drug concentration, and/or of the drug metabolite concentration, and/or of any effect directly or indirectly induced by the administration of each dose of the drug.

Such variation may be represented for example as a curve in a two-dimensional coordinate system and provides broad qualitative information regarding drug exposure of the 'simulated patient'.

Various quantitative aspects of the drug exposure of the patient may also be derived from such a curve or from its mathematical expression. For example:
- the maximum value reached by such curve indicates the maximum instantaneous drug exposure of the patient,
- conversely, the minimum value reached by such curve indicates the minimum instantaneous drug exposure of the patient,
- the amount of time between a dose administration occurrence and the subsequent local maximum of such curve is one of many indicators of the kinetics of the drug exposure of the patient, another such indicator being the amount of time between a dose administration occurrence and reaching a lower threshold value,
- the mean value of such curve across the time interval separating two consecutive dose administrations occurrences of a periodic dose regimen indicates the average drug exposure of the patient over time,
- the time integral of such curve across the time interval separating two consecutive dose administration occurrences indicates the aggregate drug exposure of the patient between these two dose administration occurrences.

The curve may also be time split, in fractions of the time interval separating two consecutive dose administration occurrences, so as to calculate the mean drug exposure of the patient during each such time split. It is for example possible, from these mean drug exposures, to determine a maximum mean drug exposure, corresponding to the maximum mean value of a time split across all time splits.

Hereafter, the expression "drug exposure of the patient" refers to data, or to a dataset, indicating one or more of the any of the above aspects of the drug exposure.

Figure 1 shows that for a set of values noted {Vd1,K1} representative of a 'simulated patient' defined as exhibiting, as individual pharmacokinetic parameters, an apparent volume of distribution Vd1 and an elimination rate constant K1 , a first drug exposure, noted DrugExp11, may be calculated for a first dose regimen noted DoseReg1 while a second drug exposure, noted DrugExp21, may be calculated for a second dose regimen noted DoseReg2.

More generally, n sets of sampled values allow calculating, for n 'simulated patients' and for m dose regimens to be tested, m sets of n calculated drug exposures each.

Each such set is statistically representative of the actual drug exposures that would be induced by administrating the drug, according to the corresponding dose regimen to be tested, to a group of hypothetical patients statistically representative of the patient population.

The calculated drug exposures, for a given dose regimen, may be compared (S5), by a comparator module, with one or more threshold values.

For example, a maximum instantaneous drug exposure may be compared with a maximum threshold value associated to an undesirable effect. This allows determining:
- whether the instantaneous drug exposure resulting of a given dose regimen remains below the maximum threshold value, which allows fully avoiding the undesirable effect,
- or, on the contrary, reaches an excessive value at some point in time.

For example, a minimum instantaneous drug exposure may be compared with a minimum threshold value associated to a lack of therapeutic effect. This allows determining:
- whether the instantaneous drug exposure resulting of a given dose regimen remains above the minimum threshold value, which allows continuously providing the therapeutic effect,
- or, on the contrary, reaches an insufficient value at some point in time.

Both comparisons above may be conducted in order to check whether the instantaneous drug exposures remain, over time, in a predetermined therapeutic window. The specific boundaries defining said therapeutic window may be predetermined for instance based on the submission report of the considered molecule and/or based on clinical trial from the literature demonstrating the patient benefit (in terms of positive effect and of toxicity) of being within said specific boundaries in terms of drug exposure.

The same principle applies with comparing maximum and minimum mean drug exposures with corresponding threshold values. This allows determining whether, for a predetermined time step, the mean drug exposure of the patient remains, across said time step, in the predetermined window.

Figure 1 shows the result of a comparison of, on the one hand, a matrix of calculated drug exposures for different 'simulated patients' and different dose regimens, and, on the other hand, a maximum threshold value. The result is shown in the form of a matrix of binary (Y/N) comparison results.

The calculated drug exposures may also each be linked (S6), or associated, by an association module, to a magnitude of a therapeutic effect, and/or to a magnitude of a undesirable effect, to be expected with said drug exposure. The association module may perform this task based on literature data that indicate the relationship between the drug exposure and the magnitude of the possible resulting effects.

Figure 1 shows the result of an association of a matrix of calculated drug exposures [DrugExp11, DrugExp12, ... DrugExp21, DrugExp22, ...] for different 'simulated patients' and different dose regimens with corresponding magnitudes of resulting effects, noted as a matrix of associated drug effects [DrugEff11, DrugEff12, ... DrugEff21, DrugEff22, ...].

Similarly, to the comparison of the calculated drug exposures, for a given dose regimen, by the comparator module, with one or more threshold values, it is also possible to compare the associated drug effect magnitudes, for a given dose regimen, with one or more threshold values.

This allows determining, for example, whether a given dose regimen would induce, for a 'simulated patient', a sufficient or an insufficient magnitude of a therapeutic effect during a specific time frame. This also allows determining, for example, whether a given dose regimen would induce, for a 'simulated patient', an acceptable or an unacceptable amount of an undesirable effect during a specific time frame.

For a given dose regimen, the calculated drug exposures in general, or the more specific results of the comparisons conducted by the comparator module, or the magnitudes of effects linked by the association module to said calculated drug exposures, may be used to determine (S7), at a scoring module, a score associated to said given dose regimen.

The score is a rating of said dose regimen. The score may be used to directly compare different dose regimens in terms of statistically meeting one or more criteria with regard to drug exposure, or to one or more resulting drug effects, for the patient population as a whole.

An example of such a criterion is whether the dose regimen to be tested successfully induces, over a signification portion of the time interval between two consecutive drug administration occurrences, a drug exposure matching the therapeutic window.

It is thus possible, according to this criterion, to determine a score indicating the rate, among the 'simulated patients', of those which calculated drug exposures match the therapeutic window.

The example provided in Figure 1 is based on such criterion. A score of "50" corresponds to half of the sets of values leading to calculated drug exposures within the therapeutic window, whereas a score of "100" corresponds to a perfect score with all the sets of values leading to calculated drug exposures within the therapeutic window.

Another example of such a criterion is the risk, within the patient population, for the dose regimen to be rested to induce an undesirable effect at a significant magnitude. It is thus possible, according to such a criterion, to determine a score indicating the rate, among the 'simulated patients', of those which calculated drug exposures are linked to a magnitude of an undesirable effect exceeding a given threshold.

Various other examples of criteria may apply, alone or in combination, depending on the specific use case, in particular depending on the exact nature of the desired therapeutic effect and of the possible undesirable effects.

It follows from the above that the calculated drug exposures may be used to determine different types of indications of a degree of compliance of the tested dose regimen with the objective of inducing, for the patient population on a statistical point of view, a drug exposure within a target range such as a therapeutic window.

These types of indications include in particular:
- the results of comparisons (S5) between the calculated drug exposures and threshold values,
- the magnitudes of effects linked (S6) to the calculated drug exposures, and
- the scores determined (S7) for each tested dose regimen.

Such indication may be used to determine a generic recommendation of one tested dose regimen over another for the patient population as a whole.

Another course of action is to consider the calculated drug exposures, or any type of indications derived from said calculated drug exposures, as a basis for individualizing a dose regimen. Individualizing a dose regimen means determining a specific, personalized, dose regimen for a specific, actual, patient.

To do so, the calculated drug exposures with a tested dose regimen may for example be processed in view of determining an expected effect on a specific patient with the tested dose regimen.

This requires acquiring data associated to said specific patient, at an input module.

For instance, a set of values associated to a specific patient for whom an appropriate dose regimen is to be determined may be obtained (S8), at the input module, for example through a human-machine interface or a machine-machine communication interface.

The set of values is indicative of a patient profile, at least to the extent of relevance for determining the expected effect of the drug on the patient with one of one or more dose regimens.

Such set of values may comprise a single value (singleton) or a plurality of values.

For example, the set of values associated to the specific patient may relate to the same parameters for which simulated values have been sampled (S2) by the sampling module, or to a subset of said parameters.

Figure 1 shows, as an example of a set of values associated to a specific patient, the set {48, F, 75} for a 48-year-old female weighing 75 kg. In this specific example, the set of values associated to a specific patient does not only relate to the same parameters for which simulated values have been sampled (S2) by the sampling module, but is also represented, for pedagogical purposes, in the same format as the sets of values formed (S3) by regrouping the simulated sampled values. This is however not a requirement. Indeed, alternately, a less informative set of values associated to the same specific patient may be {adult, female}. In yet another example, the set of values associated to the same specific patient may be {height=5'11ft, BMI=23, age=48, bloodtype=O}.

In case of discrepancy between the original format of the obtained set of values and a reference format, data conversion may of course be performed to obtain a harmonized set of values associated to the specific patient according to the reference format.

The obtained set of values associated to the specific patient may be used to determine a section of the patient population to be focused on, said section being described by the population pharmacological model in terms of variability in drug exposure and said section comprising the actual patient.

For example, if, based on the population pharmacological model, age appears to be among the main factors influencing drug exposure, then it may be relevant to focus on a section of the patient population corresponding to a similar age group.

This may be done for example:
- at the model retrieving module, by restricting the allowable range of parameter values for any parameter related to age, as a preliminary processing of the population pharmacological model, or
- at the sampling module by restricting the parameter value sampling to only output values in an allowable age range, or
- by filtering the sets of sampled parameter values to only keep the sets comprising a value related to age falling within an allowable age range around the age of the specific patient.

In an exemplary scenario, a physician may have access to data from a patient profile of a specific patient, and may seek to determine an initial dose regimen of the drug, as part of an initial prescription, for the specific patient.

In such a scenario, by granting, to the model receiving module, to the sampling module, or to the regrouping module, access to the data from the patient profile, it is possible to obtain a series of sets of sampled parameter values representing a section of the patient population having a similar profile, in terms of sources of variability with respect to drug exposure, as the specific patient.

A measurement or an observation related to an initial drug exposure of the patient with the former dose regimen may be directly obtained (S9) at the input module, for example by medical analysis. Examples include a blood concentration or a magnitude of an effect of the drug on the patient with the former dose regimen.

For example, the specific patient may already be subjected to administration of the drug according to a former dose regimen, and it may be considered to explore possibilities for adapting said dose regimen.

Measurements or observations have the advantage of accuracy, but are not a requirement to obtain the initial drug exposure of the patient.

An alternate possibility is defining input parameters of the population pharmacological model based on the set of values associated to the specific patient and further based on a set of values related to the former dose regimen. For example, the former dose regimen may be defined by a period, which is the time interval between two drug administration occurrences, and by an amount of the drug that the specific patient receives per administration occurrence. Once the input parameters are defined, it is possible to calculate or predict the associated initial drug exposure of the patient, according to the former dose regimen, then to provide the calculated or predicted initial drug exposure to the input module.

For one or more dose regimens, one or more aspects of one or more effects of the drug on the specific patient may be determined (S10), at an effect determination module, based on at least part of the calculated drug exposures.

Determining said effect for a dose regimen may specifically refer to:
- determining a risk and/or a possible magnitude of an undesirable effect for the specific patient with said dose regimen, and/or
- determining a probability of providing a therapeutic effect with said dose regimen, and/or
- determining a probability of reaching at least a target magnitude of said therapeutic effect with said dose regimen,
- determining an expected duration of said effect for the specific patient, following an administration to said patient of a dose of the drug according to the considered dose regimen, and/or
- determining any other aspect of the effect expected to be encountered by the specific patient with said dose regimen.

It is hereafter followed through with the exemplary scenario where a physician seeks to determine an initial dose regimen of the drug, as part of an initial prescription, for a specific patient.

In such a scenario, it is considered that a series of sets of sampled parameter values is obtained, these sets of values representing a section of the patient population having a similar profile, in terms of sources of variability with respect to drug exposure, as the specific patient.

For each of these sets of values, inherently representing a 'simulated patient', the drug exposure calculator module calculates (S4), for any number of possible dose regimens, a corresponding drug exposure of the 'simulated patient'. As a result, the calculated drug exposures indicate, for each of one or more tested dose regimens, the distribution in drug exposure among a section of the patient population having a similar profile, in terms of sources of variability with respect to drug exposure, as the specific patient.

Based on the calculated drug exposures for a dose regimen, it is possible to determine a probability for meeting the therapeutic window for the specific patient with said dose regimen.

For example, the calculated drug exposures for said dose regimen may each be compared (S5) by the comparator module to a lower and to a higher threshold identified as boundaries of the therapeutic window. The outcome of both comparisons may lead, for example, the comparator module to output, for each calculated drug exposure, either a first value indicating a match with the therapeutic window or a second value indicating an absence of such a match. The outputs of the comparator module may then be used downstream by the scoring module to determine (S7) a score associated to said dose regimen. For example, if for said dose regimen, the comparator module has output 96-100% of 'first values' as a result of the comparisons of each calculated drug exposure with the threshold values, then said dose regimen may be associated to a score 'A'. If 91-95% of 'first values' are obtained, then the dose regimen may be associated to a score 'B', and so on.

As a result, the effect determination module may determine (S10), as an example of an effect of the drug on the specific patient, an information in the form of the above score and of an indication that a score 'A' is indicative of a high probability for meeting the therapeutic window for the specific patient with said dose regimen, while a score 'B' is indicative of a lower probability for meeting the therapeutic window for the specific patient with said dose regimen, and so on.

Based on the calculated drug exposures for a dose regimen, it is also possible to determine the risk, with said dose regimen, that an undesirable effect occurs for the specific patient, with a magnitude exceeding a predetermined level. For example, each calculated drug exposure may be linked (S6) to an associated magnitude of the undesirable effect, based on literature data. As a result, the effect determination module may determine (S10), as another example of an effect of the drug on the specific patient, an information in the form of a proportion of the associated magnitudes exceeding said predetermined level.

In another exemplary scenario, a physician may seek to modify a former dose regimen of the drug that had been initially prescribed to a specific patient. Reasons may include diminishing the risk and/or the magnitude of an undesirable effect, increasing the probability of occurrence and/or the magnitude of a therapeutic effect, accelerating or decelerating the duration of one of said effects, etc.

The former dose regimen may be considered as a reference dose regimen.

As such, it is possible to form (S3) sets of values that are representative of the patient population, then to calculate (S4) the drug exposures, for each set of values, with the former, reference dose regimen. It is further possible to calculate (S4) the drug exposures, for each set of values, with a new, tested, dose regimen.

By calculating the drug exposures is understood determining one or more relevant aspects (in terms of probability of occurrence and/or magnitude and/or duration etc.) of the drug exposure for the sought purpose.

Each calculated drug exposure may further be linked to a corresponding drug effect.

For example:
- a drug exposure below the therapeutic window may be associated to an absence of therapeutic effect,
- a duration, after administration of a dose of the drug, before exceeding the lower boundary of the therapeutic window may be associated to a duration before providing the therapeutic effect.

For each set of values representing a corresponding 'simulated patient', it is possible to determine an absolute or relative variation of drug effect between:
- the drug effect linked to the drug exposure calculated with the reference dose regimen, and
- the drug effect linked to the drug exposure calculated with the new tested dose regimen.

Such absolute or relative variations of drug effect may be processed by the effect determination module to determine (S10), as yet another example of an effect of the drug on the specific patient, a mean absolute or relative variation of the drug effect between the former dose regimen and the tested dose regimen among the patient population, or a standard deviation of such absolute or relative variations.

The determined effect, or effects, of the drug on the specific patient with one or more dose regimens may then be provided (S11) through an interface module, for example displayed, printed or transmitted to a remote device for allowing further access and consultation along with the medical record of the specific patient.

At this stage, if several dose regimens have been tested, it is possible, for instance, to provide a list of all tested dose regimens along with the corresponding determined effect of the drug on the specific patient.

An emphasis may be put on one or more dose regimens.

For example, dose regimens may be highlighted if the risk of an undesirable effect for the patient is deemed too high, such as greater than a threshold value, to serve as an alert.

For example, the determined effect of the drug on the specific patient for a dose regimen may be indicated by a score, and the list of tested dose regimens may be automatically ordered from the best score to the worst score.

## Claims

1. A method for determining an effect of a drug on at least one patient, with at least one specific dose regimen, the method comprising:
- obtaining (S1) a population pharmacological model, said model describing a variability in drug exposure within a patient population receiving known doses of said drug,
- for at least one parameter relating to a source of said variability within the patient population, sampling (S2) a plurality of values of said parameter from a distribution conditional on individual patient characteristics and/or at least one measurement related to a drug exposure of the at least one patient,
- forming (S3) sets of values, one set of values comprising one sampled value of each of said parameter,
- by using the population pharmacological model, calculating (S4), for each set of values, a corresponding drug exposure with given dose regimens, and
- determining (S10) the effect of the drug on at least one patient, with at least one specific dose regimen, based at least on the calculated drug exposures.

2. The method of claim 1,
- wherein, the patient being submitted to a former dose regimen, the method further comprises obtaining (S9) at least one measurement related to a drug exposure of the patient with the former dose regimen, and
- wherein determining (S10) the effect of the drug on the patient is further based on said measurement.

3. The method of claim 1 or 2, wherein determining the effect of the drug comprises comparing (S5) the calculated drug exposures to at least one exposure threshold.

4. The method of claim 1 or 2, wherein:
- the method further comprises linking (S6) each calculated drug exposure to a corresponding level of magnitude of said effect of the drug, and
- determining the effect of the drug, with given dose regimens, on said patient, is based on the linked levels of magnitude.

5. The method of any of claims 1 to 4, wherein determining the effect of the drug comprises determining (S7) a score representative of said effect based on the calculated drug exposures.

6. The method of any of claims 1 to 5, wherein the patient population is a selected portion of a general population.

7. The method of any of claims 1 to 6, wherein the drug exposure relates to an instantaneous exposure value.

8. The method of any of claims 1 to 7, wherein the drug exposure relates to an average or an aggregate exposure across a predetermined time interval.

9. The method of any of claims 1 to 8,
- wherein the method further comprises, for at least one parameter relating to a source of said variability within the patient population, using direct sampling from an explicit conditional distribution of values of said parameter within the patient population.

10. The method of any of claims 1 to 9,
- wherein the method further comprises, for at least one parameter relating to a source of said variability within the patient population, using a Markov Chain Monte Carlo approach applied to the pharmacological model, and
- wherein sampling the plurality of simulated values of said parameter is based on Markov chains constructed using said Markov Chain Monte Carlo approach.

11. The method of any of claims 1 to 10, further comprising, for at least one additional dose regimen:
- by using the population pharmacological model, calculating, for each set of values, a corresponding additional drug exposure with said additional dose regimen, and,
- determining the corresponding effect of the drug, with said additional dose regimen, on the patient based on the calculated additional drug exposures.

12. The method of any of claims 1 to 11, further comprising, at least for the given dose regimen, providing (S11) the corresponding determined effect in view of determining a personalized dose regimen of the drug for the patient.

13. A computer program comprising one or more stored sequence/s of instructions that is accessible to a processing unit and which, when executed by the processing unit, causes the processing unit to carry out a method according to any of claims 1 to 12.

14. A processing circuit equipped with a processing unit (1) operably connected to a memory (2), the processing circuit being configured to carry out a method according to any of claims 1 to 12.
